(19) Europäisches Patentamt
European Patent Office
Office européen des brevets

(11) EP 4 090 311 B1

(12) **EUROPEAN PATENT SPECIFICATION**

(45) Date of publication and mention of the grant of the patent:
**22.11.2023 Bulletin 2023/47**

(21) Application number: **21701916.5**

(22) Date of filing: **14.01.2021**

(51) International Patent Classification (IPC):
*A61K 8/49* (2006.01) *A61K 8/9789* (2017.01)
*A61P 31/02* (2006.01) *A61Q 5/00* (2006.01)
*A61Q 5/02* (2006.01) *A61Q 5/12* (2006.01)
*A61Q 17/00* (2006.01) *A61Q 19/10* (2006.01)
*A61P 17/10* (2006.01)

(52) Cooperative Patent Classification (CPC):
**A61K 8/4926; A61K 8/4973; A61K 8/9789; A61P 17/10; A61P 31/02; A61Q 5/006; A61Q 5/02; A61Q 5/12; A61Q 17/005; A61Q 19/10**

(86) International application number:
**PCT/EP2021/050616**

(87) International publication number:
**WO 2021/144326 (22.07.2021 Gazette 2021/29)**

(54) **TOPICAL COMPOSITION COMPRISING HYDROXAMIC ACID AND ATRACTYLENOLIDE**

TOPISCHE ZUSAMMENSETZUNG MIT HYDROXAMSÄURE UND ATRACTYLENOLID

COMPOSITION TOPIQUE COMPRENANT DE L'ACIDE HYDROXAMIQUE ET DE L'ATRACTYLENOLIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.01.2020 PCT/CN2020/072838**
**18.02.2020 EP 20157944**

(43) Date of publication of application:
**23.11.2022 Bulletin 2022/47**

(73) Proprietors:
- **Unilever IP Holdings B.V.**
  **3013 AL Rotterdam (NL)**
  Designated Contracting States:
  **AL AT BE BG CH CZ DK EE ES FI FR GR HR HU IS LI LT LU LV MC MK NL NO PL PT RO SE SI SK SM**
- **UNILEVER GLOBAL IP LIMITED**
  **Wirral**
  **Merseyside CH62 4ZD (GB)**
  Designated Contracting States:
  **CY DE GB IE IT MT RS TR**

(72) Inventors:
- **CHU, Chung-Ching**
  **Shanghai, 200335 (CN)**
- **PU, Mingming**
  **Shanghai, 200335 (CN)**
- **WANG, Zongxiu**
  **Shanghai, 200335 (CN)**

(74) Representative: **Mathai, Neenu Grace**
**Unilever Patent Group**
**Bronland 14**
**6708 WH Wageningen (NL)**

(56) References cited:
**CN-A- 106 858 067** **CN-A- 109 700 733**
**CN-A- 110 693 751** **CN-A- 110 787 069**

- **DATABASE GNPD [Online] MINTEL; 26 July 2016 (2016-07-26), anonymous: "Luxury Treatment Shampoo", XP055720751, retrieved from www.gnpd.com Database accession no. 4170491**
- **DATABASE GNPD [Online] MINTEL; 19 October 2017 (2017-10-19), anonymous: "Restructuring Youth Cream", XP055720749, retrieved from www.gnpd.com Database accession no. 5161047**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 090 311 B1

- DATABASE GNPD [Online] MINTEL; 5 April 2019 (2019-04-05), anonymous: "Anti-Ageing Face Sun Cream SPF 50", XP055720747, retrieved from www.gnpd.com Database accession no. 6456793
- Nut Koonrungsesomboon: "Therapeutic potential and pharmacological activities of Atractylodes lancea (Thunb.) DC.", Asian Pacific journal of Tropical Medicine, 20 June 2014 (2014-06-20), pages 421-428, XP055419319, DOI: 10.1016/S1995-7645(14)60069-9 Retrieved from the Internet: URL:https://ac.els-cdn.com/S19957645146006 99/1-s2.0-S1995764514600699-main.pdf?_tid= 81005f3c-ba2b-11e7-827c-00000aacb360&acdna t=1509008346_220ba38abfce4448bb977474dbe5 c a7c [retrieved on 2017-10-26]
- YINFENG ZHOU ET AL: "Evidence of Phytoalexins in Rhizome of Atractylodis Maceocephalae Koidz Infected with Sclerotium rolfsii sacc Following Treatment with the Polysaccharides of Chrysanthemum indicum", JOURNAL OF PHYTOPATHOLOGY - PHYTOPATHOLOGISCHE ZEITSCHRIFT., vol. 164, no. 10, 24 May 2016 (2016-05-24) , pages 760-767, XP055721001, DE ISSN: 0931-1785, DOI: 10.1111/jph.12496
- SIN KWAN-SEOG ET AL: "Pharmacological Activities of the Constituents of Atractylodes Rhizomes", ARCHIVES OF PHARMACAL RESEARCH, NATL. FISHERIES UNIVERSITY, PUSAN, KR, vol. 12, no. 4, 1 December 1989 (1989-12-01), pages 236-238, XP009522132, ISSN: 0253-6269, DOI: 10.1007/BF02911051
- XIE JUN ET AL: "Pharmacological effects of medicinal components of Atractylodes lancea (Thunb.) DC.", CHINESE MEDICINE, vol. 13, no. 1, 27 November 2018 (2018-11-27), XP055720755, DOI: 10.1186/s13020-018-0216-7
- DATABASE GNPD [Online] MINTEL; 19 October 2017 (2017-10-19), anonymous: "Restructuring Youth Cream", XP055720749, Database accession no. 5161047
- DATABASE GNPD [Online] MINTEL; 5 April 2019 (2019-04-05), anonymous: "Anti-Ageing Face Sun Cream SPF 50", XP055720747, Database accession no. 6456793
- DATABASE GNPD [Online] MINTEL; 26 July 2016 (2016-07-26), anonymous: "Luxury Treatment Shampoo", XP055720751, Database accession no. 4170491


Remarks:

The file contains technical information submitted after the application was filed and not included in this specification

## Description

### Field of the Invention

This invention relates to topical antimicrobial compositions, especially cosmetic compositions and more particularly to cosmetic compositions comprising actives that interact to provide synergistic antimicrobial effect and useful at least against some microbes associated with conditions of cosmetic relevance such as dandruff and acne.

### Background of the Invention

The invention relates to an antimicrobial composition useful for cleaning and/or care of any body part but especially suitable for hair, and scalp care, hand hygiene or for facial care and cleansing.

Dandruff is an issue that affects many people globally. The condition is manifested by the shedding of clumps of dead skin cells from the scalp. These are white and readily visible therefore they present an aesthetically displeasing appearance. A factor that contributes to dandruff is certain members of the *Malassezia* yeasts. To combat them, various antidandruff compositions such as shampoos are available. Usually such shampoos contain surfactants and one or more antidandruff agent. Typical antidandruff agents are metal pyrithione e.g., zinc pyrithione (ZPTO), octopirox (piroctone olamine), azole antimicrobials (e.g. climbazole), selenium sulfide and combinations thereof.

While the problem of dandruff is mitigated to a large extent through use of the above actives in such shampoos, there is a need for more efficacious compositions.

Acne, also known as Acne vulgaris, is a common skin condition that affects nearly all adolescents and adults. It has a complex etiology involving abnormal keratinization and excess sebum production. Acne usually occurs in areas rich in sebaceous glands like the face, neck and back. A bacterium *Cutibacterium acnes* (*C. acnes, formerly known* as *Propionibacterium acnes or P. acnes*) has also been implicated in occurrence of acne. Acne has been treated in many ways. Most treatments take several weeks to months before a noticeable change is seen. Benzoyl peroxide which has an antibacterial effect has been used for mild cases of acne and is also believed to prevent formation of further acne. In very severe cases of acne, antibiotics like tetracycline, erythromycin and clindamycin have been used.

XP055720751 (Mingchen Cosmetics) discloses a luxury treatment shampoo comprising piroctone olamine and Atractylodes lancea root extract. However, it does not disclose the weight ratio between piroctone olamine and Atrctylodes lancea root extract.

CN 110693751A (Zhou Le) discloses beauty creams comprising (i) capryl hydroxamic acid and (ii) Atractylodes root powder and Atractylodes chinensis or Atractylodes lancea extract. However, it does not disclose the weight ratio between capryl hydroxamic acid and root powder and Atractylodes chinensis or Atractylodes lancea extract.

CN 110787069A (Luo Xianyi) discloses face masks comprising (i) Atractylodes macrocephalae extract and (ii) capryloyl or octanoyl hydroxamic acid. However, it does not disclose the weight ratio between (i) and (ii).

CN 109700733A (Zhangjiajie Chakunyuan Biotechnology Dev Co Ltd) discloses facial mask comprising (i) Atractylodes macrocephalae extract (as part of Ecommia seed plant extract) and (ii) capryl/octyl/octanoyl hydroxamic acid. However, it does not disclose the weight ratio between (i) and (ii).

It is thus an object of the present invention to provide for a topical composition that exhibits synergistic antimicrobial activity as compared to the individual components.

### Summary of the Invention

We have determined that when a certain active which is usually found in the natural plants is formulated together with a typical antimicrobial active which is a hydroxamic acid or hydroxamic acid derivative, the combination is highly efficacious against some microbes associated with conditions like dandruff and acne. That led us to the inference that compositions comprising the abovementioned combination could have antidandruff, antiacne and general antimicrobial activity and could be suitable for use in e.g., shampoo, body and face care. The certain active referred to hereinabove is an atractylenolide compound.

Therefore, in accordance with a first aspect is disclosed a topical composition comprising:

(i) an antimicrobial active which is at least one ofcaprylhydroxamic acid or piroctone olamine; and
(ii) an atractylenolide compound; wherein weight ratio of the amount of said atractylenolide compound to that of said antimicrobial active is at least 5:1; wherein said atractylenolide compound is atractylenolide I or II

In accordance with a second aspect is disclosed a topical composition of the first aspect for use in providing antimicrobial benefit on a topical surface of a human or animal body.

In accordance with a third aspect is disclosed a non-therapeutic method of providing topical antimicrobial benefit

on a topical surface of a human or animal body comprising a step of applying a safe and effective amount of a topical antimicrobial composition of the first aspect.

**Detailed Description of the Invention**

The compositions according to the present invention may comprise a topically acceptable carrier, vehicle or diluent which can have a variety of different forms. The topically acceptable carrier should preferably be non-irritant. "Topically-acceptable" therefore means that the carrier is suitable for topical application to the skin without causing any untoward safety or toxicity concerns. In other words, these carriers are suitable for use on mammalian skin. The typical carrier can be in the form of a hydroalcoholic system (e.g. liquids and gels), an anhydrous oil or silicone based system, or an emulsion system, including, but not limited to, oil-in-water, water-in-oil, water-in-oil-in-water, and oil-in-water-in-silicone emulsions. The emulsions can cover a broad range of consistencies including thin lotions (which can also be suitable for spray or aerosol delivery), creamy lotions, light cream and heavy creams. The emulsions can also include microemulsion systems. Other suitable topical carriers include anhydrous solids and semisolids (such as gels and sticks); and aqueous based mousse systems. Nonlimiting examples of the topical carrier systems useful in the present invention are described hereinafter.

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of or "composed of." In other words, the listed steps or options need not be exhaustive. It is noted that the examples given in the description below are intended to clarify the invention and are not intended to limit the invention to those examples per se. Similarly, all percentages and ratios contained herein are weight/weight percentages unless otherwise indicated.

Except in the operating and comparative examples, or where otherwise explicitly indicated, all numbers in this description indicating amounts of material or conditions of reaction, physical properties of materials and/or use are to be understood as modified by the word "about". Unless specified otherwise, numerical ranges expressed in the format "from x to y" are understood to include x and y. When for a specific feature multiple preferred ranges are described in the format "from x to y", it is understood that all ranges combining the different endpoints are also contemplated. The various features of the present invention referred to in individual sections above apply, as appropriate, to other sections *mutatis mutandis*. Consequently, features specified in one section may be combined with features specified in other sections as appropriate. Any section headings are added for convenience only and are not intended to limit the disclosure in any way.

By a topical composition is meant a composition for external application in the form of a leave-on or wash-off format meant for cleaning or disinfecting topical areas e.g. skin and/or hair and or oral cavity of mammals, especially humans. Such a composition includes any product applied to a human body for also improving appearance, cleansing, odor control or general aesthetics. In accordance with one aspect, the compositions in accordance with the invention are rinse off compositions. Alternatively, they are leave-on compositions. The compositions of the present invention could be in the form of a liquid, lotion, cream, foam or gel, or toner, or applied with an implement or via a face mask, pad or patch. "Skin" as used herein is meant to include skin on the face and body (e.g., neck, chest, back, arms, underarms, hands, legs, buttocks and scalp). The composition of the invention is also of relevance to applications on any other external substrates of the human body other than skin e.g. hair.

By 'an antimicrobial composition' as used herein, is meant to include a composition for topical application to skin, hair and/or scalp of mammals, especially humans. Such a composition is generally applied on to the desired topical surface of the body for few seconds to up to 24 hours. When the time of application is low say of the order of a few seconds to a few minutes after which the composition is rinsed off with water or wiped away, such a composition is known as a cleansing composition or a rinse-off composition. When the composition is applied for longer time say from several minutes to up to 24 hours and washed off usually during the process of normal personal cleaning, such a composition is known as a leave-on composition. It is more preferably used for preventing or alleviating the symptoms of dandruff on the scalp and/or hair, for antiacne benefit, or for disinfecting the hand or other parts of the human body.

"Hair care composition" as used herein, is meant to Include a composition tor topical application to hair. Non-limiting examples of such compositions include leave-on hair lotions, creams, arid wash-off shampoos, conditioners, shower gels, or a toilet bar. When the composition of the invention is a hair care composition, it preferably is a wash-off composition, especially shampoo or a conditioner.

The invention relates to a topical composition comprising:

(i) an antimicrobial active which is at least one of caprylhydroxamic acid or piroctone olamine; and
(ii) an atractylenolide compound; wherein weight ratio of the amount of said atractylenolide compound to that of said antimicrobial active is at least 5:1; wherein said atractylenolide compound is atractylenolide I or II.

The antimicrobial active according to present invention is at least one of caprylhydroxamic acid or piroctone olamine. It is most preferred that the antimicrobial active is piroctone olamine.

Piroctone is a cyclic hydroxamic acid that consists of 1-hydroxypyridin-2-one bearing methyl and 2,4,4-trimethylpentyl substituents at positions 4 and 6 respectively. The CAS number is 50650-76-5 and the compound has the general formula (a) as below:

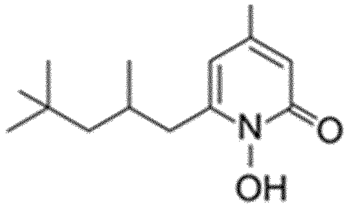

(a)

Caprylhydroxamic acid is an amino acid derived from coconut oil. It is a preservative and broad spectrum antifungal agent. The CAS number is 7377-03-9 and the compound has the general formula (b) as below:

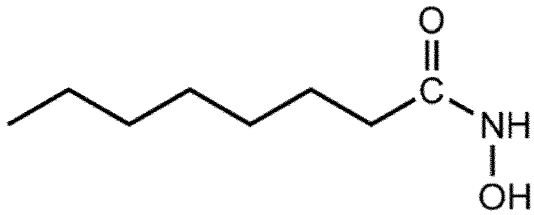

(b)

Benzohydroxamic acid is one of hydroxamic acids. The CAS number is 495-18-1 and the compound has the general formula (c) as below:

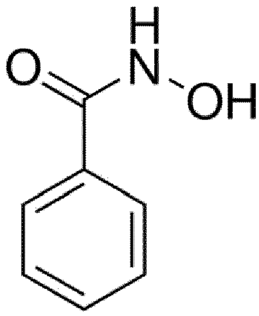

(c)

Piroctone Olamine is an olamine salt of the hydroxamic acid derivative piroctone which is a typical antimicrobial active. It is commonly known as piroctone ethanolamine with the trade name Octopirox®.

The piroctone olamine according to the present invention is a 1:1 compound of 1-hydroxy-4-methyl-6-(2,4,4-trimethylpentyl)-2(1*H*)-pyridinone with 2-aminoethanol and is also designated 1-hydroxy-4-methyl-6-(2,4,4-trimethylpentyl)-2(1*H*) pyridinone monoethanolamine salt. The CAS number is 68890-66-4 and the compound has the general formula (d) as below:

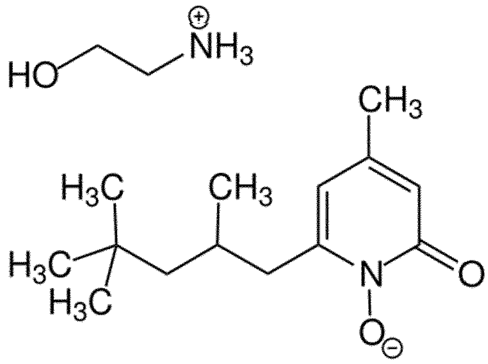

(d)

Amount of the antimicrobial active which is at least one of caprylhydroxamic acid or piroctone olamine in the composition of the invention would depend on the type of the topical composition and the precise nature of other antimicrobial actives used. It is preferred that the composition comprises 0.01 to 10 wt% of said antimicrobial active, more preferably 0.1 to 5 wt%, furthermore preferably 0.5 to 3 wt% by weight of the composition.

Atractylenolide compound

The composition in accordance with the invention comprises an atractylenolide compound. Atractylenolide compound is a eudesmane-type sesquiterpenoid lactone which can be found in *Atractylodes,* including atractylenolide I, atractylenolide II, atractylenolide III, atractylenolide IV and 4(R),15-epoxy-8β-hydroxyatractylenolide II. The expression 'an atractylenolide compound' is used to indicate the presence of one of more compounds belonging to a generalized class of compounds having the basic structural formula of eudesmane-type sesquiterpenoid lactone which can be found in atractylodes. Such atractylenolide compounds could also be found in plants-*Sarcandra glabra* or *Chloranthus serratus.*

It is preferred that the amount of the atractylenolide compound in the compositions of the invention is from 0.01 to 10 wt%. More preferably the amount is 0.01 to 5% by weight of the composition and most preferably 0.1 to 2%. When two or more atractylenolide compounds are present in the compositions of the invention, their total amount is as disclosed above.

The atractylenolide compound according to present invention is atractylenolide I (CAS 73069-13-3) or atractylenolide II (CAS 73069-14-4).

The atractylenolide compound I, II and III have the corresponding general formula (I, II and III) as below:

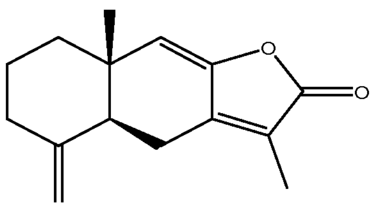

(I)

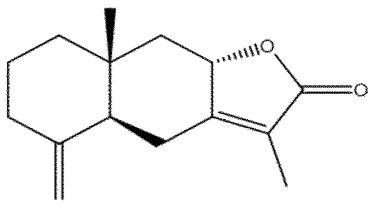

(II)

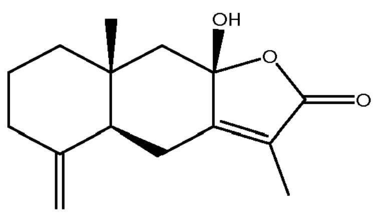

(III)

The atractylenolide compound according to present invention is at least one of atractylenolide I or atractylenolide II. Alternatively, and further preferably the composition comprises atractylenolide I and atractylenolide II. It is preferred that the composition comprises 0.01 to 10 wt% of said atractylenolide I or 0.01 to 10 wt% of said atractylenolide II, provided that when said composition comprises both atractylenolide I and atractylenolide II, then their combined amount is 0.01 to 10 wt% of said composition.

Preferably the composition of the invention comprises extract of the *Atractylodes spp,* alternatively extract of Sarcandra glabra or Chloranthus serratus, which in turn comprises said atractylenolide compound. A measured amount of the extract is chosen so that the requisite amount of the atractylenolide compound contained therein get included in the composition.

In general, *Atractylodes* is a genus in the plant family *Asteraceae.* Extract of the *Atractylodes* can be obtained from species within the Atractylodes genus. Non-limiting examples of these species include *Atractylodes amurensis*, *Atractylodes lancea, Atractylodes carlinoides, Atractylodes macrocephala, Atractylodes japonica, Atractylodes koreana, Atractylodes ovata, Atractylodes chinensis.*

*Atractylodes* extracts containing atractylenolide compound are commercially available from a variety of different sources. For example, an extract of the root of *Atractylodes macrocephala* which comprises both atractylenolide I and atractylenolide II, is available from Hangzhou Liaoyuan. Individual atractylenolide I and atractylenolide II are available from MCE. Alternatively, a person of ordinary skill in the art would be able to isolate Atractylodes extract from the Atractylodes root by using any suitable isolation and purification method known in the art. In some embodiments, the extract of atractylodes may be obtained from dried *Atractylodes* plant root and can be prepared by any means known or to be developed in the art.

As the compositions comprise piroctone olamine and an atractylenolide compound, a ratio is maintained between the amount of the active ingredients to get the efficacy. The weight ratio of the amount of said atractylenolide compound to said antimicrobial active is at least 5:1. Preferably the weight ratio of the amount of said atractylenolide compound to said antimicrobial active is from 5:1 to 1280:1, more preferably 5:1 to 100:1, more preferably from 5:1 to 50:1, even more preferably 5:1 to 40:1, furthermore preferably from 5:1 to 20:1, particularly preferably from 5:1 to 10:1.

The sum of the fractional inhibitory concentrations (ΣFIC) is widely used in the context of combinations of antimicrobial ingredients. It is a tool to determine whether the antimicrobial ingredients (when used in combination) have synergistic effect or antagonistic effect or neither of the two, i.e., an additive effect. The present inventors have resorted to the ΣFIC test to evaluate the combined effect of antimicrobial active with atractylenolide I or atractylenolide II against *M. furfur.*

It is believed that while the antimicrobial active and atractylenolide compound according to the present invention present in the compositions of the invention, the atractylenolide compound interacts synergistically with the antimicrobial active to make it even more efficacious.

It is preferred that the topical composition of the invention comprises piroctone olamine and atractylenolide I. In this case, the weight ratio of the amount of atractylenolide I to that of piroctone olamine is at least 5:1. In this case it is preferred that weight ratio of the amount of atractylenolide I to that of piroctone olamine is 5:1 to 1280:1, alternatively 5:1 to 100:1, alternatively 5:1 to 50:1, alternatively 5:1 to 40:1 and further alternatively 5:1 to 10:1.

Alternatively, the composition comprises piroctone olamine and atractylenolide II. In this case, the weight ratio of the amount of atractylenolide II to that of piroctone olamine is at least 5:1. In this case it is preferred that the weight ratio of the amount of atractylenolide II to that of piroctone olamine is 5:1 to 2560:1, alternatively5:1 to 100:1, alternatively 5:1 to 80:1, alternatively 5:1 to 50:1, and further alternatively 5:1 to 10:1.

It is preferred that the topical composition of the invention comprises caprylhydroxamic acid and atractylenolide I. In this case, the weight ratio of the amount of atractylenolide I to that of caprylhydroxamic acid is at least 5:1. In this case it is preferred that weight ratio of the amount of atractylenolide I to that of caprylhydroxamic acid is 5:1 to 1280:1, alternatively5:1 to 100:1, alternatively 5:1 to 50:1, alternatively 5:1 to 40:1 and further alternatively 5:1 to 10:1.

Alternatively, the composition comprises caprylhydroxamic acid and atractylenolide II. In this case, the weight ratio of the amount of atractylenolide II to that of caprylhydroxamic acid is at least 5:1. In this case it is preferred that the weight ratio of the amount of atractylenolide II to that of caprylhydroxamic acid is 5:1 to 2560:1; alternatively5:1 to 100:1, alternatively 5:1 to 80:1, alternatively 5:1 to 50:1, and further alternatively 5:1 to 10:1.

<u>Other ingredients</u>

The composition of the invention preferably comprises a cosmetically acceptable vehicle. The cosmetically acceptable vehicle is such that the composition can be prepared, e.g., as a shampoo, conditioner, body wash, hand wash or face wash product, cream, lotion, gel, powder, ointment, hand sanitiser or a soap bar and the rest of the ingredients would vary accordingly.

In one aspect the topical compositions in accordance with the invention is a hair care composition. Preferably such a composition is a shampoo, a hair conditioner, a hair serum or a hair oil. Most preferably the topical composition

is an antidandruff composition effective against at least some *Malessezia spp.*

When the composition of the invention is a shampoo, it preferably comprises other ingredients which are generally included in such compositions.

A shampoo preferably comprises 1 to 20 wt%, more preferably 2 to 16 wt%, furthermore preferably from 3 to 16 wt% anionic surfactants, e.g. an alkyl sulphate and/or ethoxylated alkyl sulfate surfactant. Preferred alkyl sulfates are $C_{8-18}$ alkyl sulfates, more preferably $C_{12-18}$ alkyl sulfates, preferably in the form of a salt with a solubilising cation such as sodium, potassium, ammonium or substituted ammonium.

Particulartly preferred alkyl ether sulfates are those having the formula: $RO(CH_2CH_2O)_nSO_3M$; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulfate (SLES). SLES having an average degree of ethoxylation of from 0.5 to 3, preferably 1 to 3 is especially preferred.

Shampoo compositions according to the invention may comprise one or more further anionic cleansing surfactants which are cosmetically acceptable and suitable for topical application to the hair. Examples include the alkaryl sulphonates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphosuccinates, alkyl ether phosphates and alkyl ether carboxylic acids and salts thereof may contain from 1 to 20 ethylene oxide or propylene oxide units per molecule.

Typically, suitable anionic surfactants include sodium oleyl succinate, ammonium lauryl sulphosuccinate, sodium lauryl ether sulphosuccinate, sodium dodecylbenzene sulphonate, triethanolamine dodecylbenzene sulphonate, lauryl ether carboxylic acid and sodium N-lauryl sarcosinate. Suitable preferred additional anionic cleansing surfactants are sodium lauryl ether sulphosuccinate(n)EO, (where n is from 1 to 3), lauryl ether carboxylic acid (n) EO (where n is from 10 to 20).

Mixtures of any of the foregoing anionic cleansing surfactants may also be suitable. The shampoo composition of the invention preferably additionally comprises 0.1 to 10 wt%, more preferably from 0.5 to 8 wt% of an amphoteric surfactant, preferably a betaine surfactant such as alkyl amidopropyl betaine surfactant, for example cocoamidopropyl betaine.

The pH of the composition is preferably equal to or higher than 4.0, more preferably in the range of 5.0 to 10.0.

It is preferred that the shampoo composition additionally comprises 0.1 to 3 wt%, more preferably 0.1 to 1.5 wt% of a zinc compound. The presence of zinc in the composition is believed to improve the antidandruff efficacy. Suitable zinc compounds are ZPTO, zinc oxide, zinc citrate, zinc malonate, zinc carbonate or a combination thereof.

Preferably the shampoo composition additionally comprises 0.01 to 2 wt%, more preferably 0.025 to 0.75 wt% conazole fungicide. Preferably the conazole fungicide is ketoconazole or climbazole or mixture thereof. The presence of a conazole fungicide is believed to improve the deposition of zinc pyrithione (ZPTO).

The shampoo composition further preferably comprises a suspending agent. Suitable suspending agents are polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol distearate are preferred long chain acyl derivatives, since these impart pearlescence to the composition. Polyacrylic acid is available commercially as Carbopol® 420, Carbopol® 488 or Carbopol® 493. Polymers of acrylic acid cross-linked with a polyfunctional agent may also be used; they are available commercially as Carbopol® 910, Carbopol® 934, Carbopol® 941 and Carbopol® 980. An example of a suitable copolymer of a carboxylic acid containing monomer and acrylic acid esters is Carbopol® 1342. All Carbopol (trademark) materials are available from Goodrich.

Suitable cross-linked polymers of acrylic acid and acrylate esters are Pemulen® TR1 and Pemulen® TR2. A suitable hetero polysaccharide gum is xanthan gum, for example that available as Kelzan.

Mixtures of any of the above suspending agents may be used. Preferred is a mixture of cross-linked polymer of acrylic acid and crystalline long chain acyl derivative.

Suspending agent, if included, will generally be present at 0.1 to 10 wt%, preferably from 0.5 to 6 wt%.

A composition of the invention may contain other ingredients for enhancing performance and/or consumer acceptability. Such ingredients include fragrance, dyes and pigments, pH adjusting agents, pearlisers or opacifiers, viscosity modifiers, preservatives, and natural hair nutrients such as botanicals, fruit extracts, sugar derivatives and amino acids.

It is preferred that the shampoo composition is aqueous based. It preferably comprises 70 to 95 wt% water.

Hair Conditioner

As an alternative, the topical composition of the invention is a hair conditioner.

When conditioning benefits are to be delivered through the composition of the invention the composition is called a hair conditioner. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. An especially useful conditioning agent is a silicone, preferably a non-volatile silicone. Advantageously compositions herein may include one or more silicones. The silicones are conditioning agents found in dispersed or suspended particulate form. They are intended to deposit onto hair remaining behind after rinsing of the hair with water. Suitable silicone oils may include polyalkyl siloxanes, polyaryl siloxanes, polyalkylaryl siloxanes, polyether siloxane copolymers and mixtures thereof. Amino silicones are often formulated with shampoo compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/Dimethicone Crosspolymers (e.g. Dow Corning 9040 and 9041).

It is preferred that hair conditioner composition of the invention comprises 0.1 to 10 wt%, more preferably from about 0.1 to about 8 wt% silicone. Alternatively, the hair conditioner is silicone-free, containing not more than 1 wt% silicone. It is preferred that pH of the composition is more than 4.0, more preferably 5.0 to 7.0.

Hair conditioner composition of the invention preferably may also comprise 0.5 to 10 wt% fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

Skin cleansing

The composition of the invention may be used for skin care e.g. body or face wash. The topical composition may further comprise a surfactant. The preferred surfactants are nonionic surfactants.

Thus, in a highly preferred aspect, the topical compositions include the surfactant selected from the group of anionic surfactants.

When the surfactants are present, the topical composition preferably comprises 1 to 90% surfactant by weight of the composition.

When surfactant is used, a particularly preferred surfactant is soap. Soap is a suitable surfactant for personal washing applications of the topical composition of the invention. When present, the soap, of the present is preferably present in an amount of 1 to 90%, preferably from 10 to 85%, more preferably 25 to 75% by weight of the composition. Preferred compositions may include other known ingredients such as perfumes, pigments, preservatives, emollients, sunscreens, emulsifiers, gelling agents and thickening agents. Choice of these ingredients will largely depend on the format of the composition.

Water is a preferred carrier. When water is present, it is preferably present in at least 1%, more preferably at least 2%, furthermore preferably at least 5% by weight of the composition. When water is the carrier, a preferred liquid composition comprises 10 to 99.8% by weight water. The liquid topical composition is useful as a skin antiseptic liquid, for skin cleansing, in particular for hand wash or a face wash. When water is the carrier, a preferred solid composition comprises 5 to 30% by weight water.

The solid topical composition is preferably in form of a shaped solid, more preferably a bar. The solid topical composition is particularly useful for skin cleansing in particular for hand wash or a face wash.

According to another aspect, inorganic particulate material is also a suitable carrier. When inorganic particulate material is the carrier, the topical composition is in a solid form. Preferably the inorganic particulate material is talc. When the inorganic particulate material is talc, the solid antimicrobial composition is particularly useful as a talcum powder for application on face or body.

In another aspect of the present invention, the composition of the present invention is suitable for use in wipes for personal hygiene.

Use and Method in Accordance with the invention

In accordance with a second aspect is disclosed a topical composition of the first aspect for use in providing

antimicrobial benefit on a topical surface of a human or animal body. It also discloses a topical composition for use against at least some *Malessezia spp* on a topical surface of a human or animal body. Preferably the topical composition is an antidandruff composition effective against at least some *Malessezia spp* on a topical surface of a human or animal body. Alternatively the topical composition is an antiacne composition effective at least against *C. acnes* on a topical surface of a human or animal body. Further alternatively the topical composition is rinse-off or a leave-on composition effective at least against *S. aureus.*

In accordance with another aspect is disclosed a non-therapeutic method of providing topical antimicrobial benefit on a topical surface of a human or animal body comprising a step of applying a safe and effective amount of a topical antimicrobial composition of the first aspect. The term safe an effective amount is well known to persons skilled in the art and such amounts may vary depending on the product format, for example, the said amount in the case of a leave on composition could be 1 to 2 ml for each application, while the same amount could be 5 to 10 ml for each application in the case of shampoos. The method may be utilized for preventing or treating acne, dandruff or for maintaining general hygiene.

Preferaly the topical composition is an antidandruff composition effective against at least some *Malessezia spp.* Alternatively, the topical composition is an antiacne composition effective at least against *C. acnes.* Further alternatively the topical composition is rinse-off or a leave-on composition effective at least against *S. aureus.*

When the atractylenolide compound is atractylenolide I or II, the method is more effective when the weight ratio of the atractylenolide compound to the antimicrobial active on the *Malassezia furfur* cell is in the range of 5:1 to 1280:1, even more effective when in the range of 40:1 to 100:1.

In accordance with another aspect is disclosed a topical composition for use as an antidandruff composition effective against at least some *Malessezia spp.* In accordance with yet another aspect is disclosed a topical composition for use as an antiacne composition effective at least against *C. acnes.* In accordance with yet another aspect is disclosed a rinse-off or a leave-on composition effective at least against *S. aureus.*

The invention will now be described in detail with the help of the following non-limiting examples.

## Examples

The antimicrobial efficacy of exemplary compositions according to the invention was determined as against *M. furfur.*

The concerned procedures will now be briefly explained.

Method 1: ∑FIC assay against *M. furfur.*

Step 1: Microbe culture and preparation

*M. furfur* (CBS1878) was maintained on MD agar plate (Solution A) was cultured into 20ml of growth medium Pityrosporum Broth (PB, Solution B). They are then incubated at 32 °C for 48 hours with shaking. Then, 1ml of the first broth culture is transferred into 9ml of fresh PB and incubated at 32 °C for 48 hours with shaking. The final culture should contain 2 to $6\times10^6$ cells/ml. This is achieved by diluting using PB to $5\times10^5$ cells/ml.

Preparation of Solution A:

| | |
|---|---|
| Modified Dixon Agar (MD) | |
| 36g | Malt Extract (Oxoid) |
| 6g | Mycological Peptone (Oxoid) |
| 10 | Purified Agar (Oxoid) |
| 20g | Ox-bile (Oxoid) |
| 2ml | Oleic acid (Sigma) |
| 2ml | Glycerol (Sigma) |
| 10ml | Tween 40 (Sigma) |
| Deionized Water to 1000ml | |
| 50mg (1 vial) dissolved in 2ml 95% ethanol | Chloramphenicol (Oxoid SR078E) (Ensure stirring thoroughly including after autoclaving) |

Preparation of Solution B:

| | |
|---|---|
| Pityrosporum Broth (PB) | |
| 10 g | Bacteriological Peptone |
| 0.1 g | Yeast extract |
| 10 g | Ox-bile |
| 2.5 g | Taurocholic acid |
| 10 g | Glucose |
| 1L | Deionised water |
| 0.5 ml | Tween60 |
| 1 ml | Glycerol |
| Adjusted | pH to 6.2 |
| After sterilization | |
| 0.5 ml | UHT milk |

Step 2: In-vitro susceptibility testing

Octopirox® was serially diluted (2-fold) to prepare in a range of 60-1000 ppm in the growth medium. Testing atractylenolide compound (stock: 4 mg/ml) was 2-fold serially diluted in DMSO to prepare in a range of 40000-10 ppm. Binary combinations of octopirox and the testing compound were prepared in 96-well plate by mixing 10 μl of octopirox solution with 10 μl of testing compound. The solution in each well were further mixed with 180 μl of M. furfur strain suspensions in PB.

The final cell density in the testing plate is around $5*10^4$ cell/ml. The final concentration of each ingredient in ppm (parts per million) after (20-fold dilution) was as follows. Each of these concentrations was tried out to determine the FIC value for (either of) Octopirox® in combination with one of the atractylenolide compound. The broth medium and solvent control as taken as negative controls for comparison of results.

Octopirox® - 50, 25, 12.5, 6, 3 and 0.

Atractylenolide I - 2000, 1000, 500, 250, 125, 62.5, 31.25, 15.63, 7.8, 3.9, 2.0, 1.0, 0.5 and 0.

Atractylenolide II - 2000, 1000, 500, 250, 125, 62.5, 31.25, 15.63, 7.8, 3.9, 2.0, 1.0, 0.5 and 0.

Multi-channel pipette was used to mix the compound and strain suspension. Thereafter the 96-well plates were incubated in an incubator. In accordance with the test protocols that were followed, *M. furfur* was incubated aerobically at 32 °C for 1 day. Thereafter 20 μl alamar blue (0.1%) was dispensed in each well and the procedure of incubation (as abovementioned) was repeated. Finally, the change in colour of the indicator was monitored to check for visible signs of microbial growth or inhibition of the growth. If the colour changed to red it indicated growth (of microbes) and blue indicated no growth or inhibition of growth.

Step 3: Calculation: ΣFIC test

(1) Minimum Inhibition Concentration (MIC):

The MIC is defined as the absolute lowest concentration of active that provides complete microbial growth inhibition as indicated by the blue color of alamar blue under the tested condition.

(2) Fractional Inhibition Concentration (FIC):

The ΣFIC test was conducted based on the principle previously described in Hall MJ, Middleton RF, & Westmacott D (1983), The fractional inhibitory concentration (FIC) index as a measure of synergy. Journal of Antimicrobial Chemotherapy 11(5):427-433. The procedure is as follows:

The differing behaviours of inhibitory antimicrobials in isolation and mixtures have been widely explored using the concept of the Fractional Concentration (FC) and Fractional Inhibitory Concentration (FIC). The parameter can be defined as follows:

$$\text{FIC (component a)} = \frac{\text{MIC (component a tested in the mixture)}}{\text{MIC (component a tested as a single active)}}$$

(3) Synergy and Additivity

The interactions between antimicrobials can be additive, synergistic or antagonistic depending on whether the efficacy of the combination is equivalent to, greater than or less than that obtained for the same total concentration of the individual components when tested alone.

After all the observations were recorded and tabulated, the Fractional Inhibition Concentration (FIC) was calculated.

The combination effect of inhibitory antimicrobials is widely explored using the concept of the Fractional Concentration (FC) and Fractional Inhibitory Concentration (FIC). This parameter is defined as follows:

$$\sum \text{FIC} = \text{FIC (component 1)} + \text{FIC (component 2)}$$

Further, the inference that could be drawn from the value of ΣFIC is summarised in the table below. There is no consistent approach in the academic or patent literature in defining precise limiting ΣFIC values that differentiate synergy from additivity or antagonism. In this study, we have adopted a liberal approach defining any binary mixture with ΣFIC < 0.9 as showing evidence of synergistic behavior.

| | | |
|---|---|---|
| ΣFIC = 1 | additive antimicrobial activity | not acceptable |
| ΣFIC > 1 | antagonistic antimicrobial activity | not acceptable |
| ΣFIC < 0.9 | synergistic antimicrobial activity | inventive |

The final concentration of each ingredient in ppm (parts per million) after (10-fold dilution) was as follows. Each of these concentrations was tried out to determine the FIC value for (either of) octopirox in combination with one of the atractylenolide compound.

Results:

It was observed that there was synergistic effect of octopirox with atractylenolide compound against *M. furfur* (Table 1).

Table 1: Effect of octopirox and atractylenolide compound against M. *furfur*

| atractylenolide compound | The range of concentration of the atractylenolide compound (ppm) where the ΣFIC was less than 0.9 | The range of ratio of atractylenolide compound / octopirox (w/w) where ΣFIC was less than 0.9 |
|---|---|---|
| Atractylenolide I | 31.25-500 | 5-1280 |
| Atractylenolide II | 31.25-1000 | 5-2560 |

The data indicates a significantly wide range of concentration of the concerned atractylenolide compound that is at the disposal of formulation scientists which they could use to their advantage. The fact that ΣFIC was less than 0.9 across the broad ranges of of the atractylenolide compound when used in combination with octopirox, implies that is possible to formulate highly efficacious antimicrobial compositions comprising the two active ingredients after giving due consideration to the fact that the experiments were conducted under test conditions and the amount of the ingredients may not match or reflect the practical amounts under in-use conditions, i.e., compositions like a shampoo or a skin care cream.

Therefore, in summary, the observations tabulated in Tables 1 clearly indicate that an atractylenolide compound interacts synergistically with the antimicrobial active according to the present invention. Their interaction was found to be synergistic across broad ranges of concentration and the synergistic interaction was evident from the fact that the ΣFIC was less than 0.9.

Method 2: Zone off Inhibition (ZOI) assay

Atractylodes macrocephala (AM) root water extract (purchased from Huzhou Liaoyuan, China) was prepared at concentration of 0.75%, and 1.5% in 50% water/DMSO. Octopirox (OCT) was prepared at concentration of 0.1% and 0.2% in DMSO. Prepare Malassezia growth agar plates, and then spread 200 μL *M. furfur* ($10^6$ CFU) (CBS 1878) on to the agar. Several sterile filter papers (diameter=6mm) were placed on the agar plate. 10μL of compound solutions were added on the filter paper. The plates were Incubate at 32 °C for 2 days. Clear growth inhibition zone was recorded.

Calculation formula:

Synergistic antimicrobial activity: Inhibition distance for compound1+compound2 combination > Inhibition distance for compound 1 alone + Inhibition distance for compound 2 alone

Results:

It was observed that there was synergistic effect of octopirox with atractylodes macrocephala (AM) root water extract against *M. furfur* (Table 2).

Table 2: Effect of octopirox and atractylodes macrocephala (AM) root water extract against ***M. furfur***

| Compound | Inhibition distance(mm)= (inhibition zone diameter - filter paper diameter) / 2 | Stdev | Synergistic antimicrobial activity |
|---|---|---|---|
| 1.5% AM | 2.1 | 0.04 | |
| 0.1% OCT | 2.7 | 0.00 | |
| 1.5% AM+0.1%OCT | 5.1 | 0.18 | Yes |
| 0.75%AM | 1.3 | 0.25 | |
| 0.1%OCT | 2.7 | 0.42 | |
| 0.75%AM+0.1%OCT | 5.9 | 0.46 | Yes |

The observations tabulated in Tables 2 clearly indicate that the atractylenolide compound which is in form of atractylodes macrocephala root water extract interacts synergistically with octopirox. It indicates that an atractylenolide compound interacts synergistically with the antimicrobial active according to the present invention.

Combination of Octopirox® with other known antimicrobial active ZPTO.

Invitro ΣFIC assay against *M. furfur.* similar to that used above was used to carry out experiments to determine if the interaction between Octopirox® and other well-known antimicrobial active like zinc pyrithione yields synergistic antimicrobial activity. The experiments were carried out over a concentration range of ingredients as below: Octopirox® - 50, 25, 12.5, 6, 3 and 0.

ZnPT: 50, 25, 12.5, 6.25, 3.13, 1.56, 0.78, 0.39, 0.2, 0.1, 0.05 and 0.

The ΣFIC assay was carried out and the data indicates that the Octopirox® cannot interact synergistically with ZPTO to yield synergistic antimicrobial activity. Thus, one cannot conclude that combinations of piroctone olamine with any known antimicrobial active will always yield synergies. Surprisingly, the combination of the atractylenolide compound with the antimicrobial active claimed in the present invention displays synergistic behaviour.

All the experiments disclosed hereinabove were conducted under in vitro conditions to ascertain whether the combination of a specified antimicrobial active - piroctone olamine and an atractylenolide compound was synergistic, additive or antagonistic visa-vis their individual activity against the concerned microbe. As far as the experiments were concerned, the concentrations of the ingredients were chosen to fall within the allowable limits permitted by the concerned tests and in which it was possible to record the technical effects. Therefore, the concentrations at which the tests were conducted might not appear to fall within the range in which such ingredients are generally used in cosmetic compositions (usually in wt%).

The compositions of the invention may be formulated as an emulsion or a gel with other usual ingredients which may affect the concentration of the desired actives in the oil phase and in the water phase. Such compositions might also have a different set of physical and hydrodynamic properties like partition coefficients, diffusional rates, convective transport rates and rheological properties. Therefore, it is expected that the concentrations to be used when formulated

as a composition could be different from that at the cellular levels at which the experiments were carried out and usually the in-use concentrations are orders of magnitude higher.

Impact of ratio between of Octopirox® and Atractylenolide compound on the inhibition efficacy.

Composition contains Octopirox® and Atractylenolide compound were prepared as shown in Table 3 and Table 4. The solution was diluted with the culture growth medium by 8-fold, and further mixed with 9 volumes of *M. furfur* suspensions in PB to observe Malassezia growth difference as the assay request. Same assay was used as showed above. The mixture was incubated at 32°C for 48 hours to monitor Malassezia growth. The observed results are summarized in the Table 3 and Table 4. Since FIC is defined as the fraction inhibition concentration, thus the ΣFIC is reported here for those composition that inhibited the growth of Malassezia, but not available for those with no inhibition efficacy.

Table 3: Impact of ratio between Octopirox® **and atractylenolide I against *M. furfur***

| Example | Octopirox® (%) | Atractylenolide I (%) | Ratio of Atractylenolide I / Octopirox® | Inhibition (Yes/No) | Σ FIC |
|---|---|---|---|---|---|
| A | 0 | 0 | - | No | - |
| B | 0 | 0.13 | - | No | - |
| C | 0 | 0.25 | - | No | - |
| D | 0 | 2 | - | No | - |
| E | 0 | 4 | - | No | - |
| F | 0.003 | 0 | - | No | - |
| G | 0.05 | 0 | - | No | - |
| H | 0.05 | 0.13 | 2.5 | No | - |
| 1 | 0.05 | 0.25 | 5 | Yes | 0.5 |
| 2 | 0.05 | 2 | 40 | Yes | 0.8 |
| 3 | 0.003 | 4 | 1280 | Yes | 0.5 |

The observations tabulated in Tables 3 clearly indicate that the atractylenolide I interacts synergistically with piroctone olamine for inhibiting the growth of *M. furfur* (Σ FIC<0.9) only when the weight ratio between atractylenolide I and piroctone olamine is at least 5:1 (Examples 1-3).

Table 4: Impact of ratio between Octopirox® **and atractylenolide II against M. *furfur***

| Example | Octopirox® (%) | Atractylenolide II (%) | Ratio of Atractylenolide I / Octopirox® | Inhibition (Yes/No) | Σ FIC |
|---|---|---|---|---|---|
| I | 0 | 0 | - | No | - |
| J | 0 | 0.13 | - | No | - |
| K | 0 | 0.25 | - | No | - |
| L | 0 | 4 | - | No | - |
| M | 0 | 8 | - | No | - |
| N | 0.003 | 0 | - | No | - |
| O | 0.05 | 0 | - | No | - |
| P | 0.05 | 0.13 | 2.5 | No | - |
| 4 | 0.05 | 0.25 | 5 | Yes | 0.5 |
| 5 | 0.05 | 4 | 80 | Yes | 0.8 |
| 6 | 0.003 | 8 | 2560 | Yes | 0.5 |

The observations tabulated in Tables 4 clearly indicate that the atractylenolide II interacts synergistically with piroctone olamine for inhibiting the growth of *M. furfur* (Σ FIC<0.9) only when the weight ratio between atractylenolide II and piroctone olamine is at least 5:1 (Examples 4-6).

## Claims

1. A topical composition comprising:

   (i) an antimicrobial active which is at least one of caprylhydroxamic acid or piroctone olamine; and
   (ii) an atractylenolide compound; wherein weight ratio of the amount of said atractylenolide compound to that of said antimicrobial active is at least 5:1; wherein said atractylenolide compound is atractylenolide I or II.

2. A composition as claimed in claim 1 wherein weight ratio of the amount of said atractylenolide compound to that of said antimicrobial active is from 5:1 to 1280:1.

3. A composition as claimed in claim 1 or 2 comprising extract of *Atractylodes spp*, alternatively extract of Sarcandra glabra or Chloranthus serratus, which in turn comprises said atractylenolide compound.

4. A composition as claimed in any of claims 1 to 3 wherein the amount of said atractylenolide compound is 0.01 to 10 wt%.

5. A composition as claimed in any of claims 1 to 4 wherein the amount of said antimicrobial active is 0.01 to 10 wt%.

6. A composition as claimed in any of claims 1 to 5 wherein the composition comprises a cosmetically acceptable carrier comprising water.

7. A composition as claimed in any of claims 1 to 6 wherein the carrier additionally comprises a surfactant.

8. A composition as claimed in any of claims 1 to 7 wherein the composition is a wash-off or leave-on hair care composition.

9. A topical composition as claimed in any of claims 1 to 8 for use in providing antimicrobial benefit on a topical surface of a human or animal body.

10. A topical composition for use as claimed in claim 9 for use against at least some *Malassezia spp* on a topical surface of a human or animal body.

11. A non-therapeutic method of providing topical antimicrobial benefit on a topical surface of a human or animal body comprising a step of applying a safe and effective amount of a topical antimicrobial composition as claimed in any of claims 1 to 8.

## Patentansprüche

1. Topische Zusammensetzung, umfassend:

   (i) einen antimikrobiellen Wirkstoff, der mindestens eine der folgenden Substanzen ist: Caprylhydroxamsäure oder Piroctón-Olamin; und
   (ii) eine Atractylenolid-Verbindung; wobei das Gewichtsverhältnis der Menge der Atractylenolid-Verbindung zu der des antimikrobiellen Wirkstoffs mindestens 5:1 beträgt; wobei die Atractylenolid-Verbindung Atractylenolid I oder II ist.

2. Zusammensetzung, wie im Anspruch 1 beansprucht, wobei das Gewichtsverhältnis der Menge der Atractylenolid-Verbindung zu der des antimikrobiellen Wirkstoffs 5:1 bis 1280:1 beträgt.

3. Zusammensetzung, wie im Anspruch 1 oder 2 beansprucht, umfassend einen Extrakt von *Atractylodes spp,* alternativ einen Extrakt von *Sarcandra glabra* oder *Chloranthus serratus,* der wiederum die Atractylenolid-Verbindung umfasst.

4. Zusammensetzung, wie in einem der Ansprüche 1 bis 3 beansprucht, wobei die Menge der Atractylenolid-Verbindung 0,01 bis 10 Gew.-% beträgt.

5. Zusammensetzung, wie in einem der Ansprüche 1 bis 4 beansprucht, wobei die Menge des antimikrobiellen Wirkstoffs 0,01 bis 10 Gew.-% beträgt.

6. Zusammensetzung, wie in einem der Ansprüche 1 bis 5 beansprucht, wobei die Zusammensetzung einen kosmetisch akzeptablen Träger, umfassend Wasser, umfasst.

7. Zusammensetzung, wie in einem der Ansprüche 1 bis 6 beansprucht, wobei der Träger zusätzlich ein Tensid umfasst.

8. Zusammensetzung, wie in einem der Ansprüche 1 bis 7 beansprucht, wobei die Zusammensetzung eine abwaschbare oder Leave-on-Haarpflegezusammensetzung ist.

9. Topische Zusammensetzung, wie in einem der Ansprüche 1 bis 8 beansprucht, zur Verwendung bei der Bereitstellung einer antimikrobiellen Wirkung auf einer topischen Oberfläche eines menschlichen oder tierischen Körpers.

10. Topische Zusammensetzung, zur Verwendung wie im Anspruch 9 beansprucht, gegen mindestens einige *Malassezia spp* auf einer topischen Oberfläche eines menschlichen oder tierischen Körpers.

11. Nicht-therapeutisches Verfahren zum Bereitstellen einer topischen antimikrobiellen Wirkung auf einer topischen Oberfläche eines menschlichen oder tierischen Körpers, umfassend einen Schritt des Auftragens einer sicheren und wirksamen Menge einer topischen antimikrobiellen Zusammensetzung, wie in einem der Ansprüche 1 bis 8 beansprucht.

**Revendications**

1. Composition topique comprenant:

    (i) un actif antimicrobien qui est au moins l'un de l'acide caprylhydroxamique ou de la piroctone olamine; et
    (ii) un composé atractylénolide; où le rapport pondéral de la quantité dudit composé atractylénolide à celle dudit actif antimicrobien est d'au moins 5:1; où ledit composé atractylénolide est l'atractylénolide I ou II.

2. Composition selon la revendication 1, où le rapport pondéral de la quantité dudit composé atractylénolide à celle dudit actif antimicrobien est de 5:1 à 1280:1.

3. Composition selon la revendication 1 ou 2, comprenant un extrait d *Atractylodes spp*, ou à titre d'alternative un extrait de Sarcandra glabra ou de Chloranthus serratus, qui comprend à son tour ledit composé atractylénolide.

4. Composition selon l'une quelconque des revendications 1 à 3, où la quantité dudit composé atractylénolide est de 0,01 à 10 % en poids.

5. Composition selon l'une quelconque des revendications 1 à 4, où la quantité dudit actif antimicrobien est de 0,01 à 10 % en poids.

6. Composition selon l'une quelconque des revendications 1 à 5, où la composition comprend un vecteur acceptable du point de vue cosmétique comprenant de l'eau.

7. Composition selon l'une quelconque des revendications 1 à 6, où le vecteur comprend en outre un tensioactif.

8. Composition selon l'une quelconque des revendications 1 à 7, où la composition est une composition de soins capillaires à rincer ou à laisser appliquer.

9. Composition topique selon l'une quelconque des revendications 1 à 8, destinée à être utilisée pour procurer un bénéfice antimicrobien sur une surface topique d'un corps humain ou animal.

10. Composition topique destinée à être utilisée selon la revendication 9, destinée à être utilisée contre au moins certains

*Malassezia spp* sur une surface topique d'un corps humain ou animal.

11. Procédé non thérapeutique pour procurer un bénéfice antimicrobien topique sur une surface topique d'un corps humain ou animal comprenant une étape d'application d'une quantité inoffensive et efficace d'une composition antimicrobienne topique telle que revendiquée dans l'une quelconque des revendications 1 à 8.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 110693751 A, Zhou Le
- CN 110787069 A, Luo Xianyi
- CN 109700733 A


### Non-patent literature cited in the description


- *CHEMICAL ABSTRACTS,* 50650-76-5
- *CHEMICAL ABSTRACTS,* 7377-03-9
- *CHEMICAL ABSTRACTS,* 495-18-1
- *CHEMICAL ABSTRACTS,* 68890-66-4
- *CHEMICAL ABSTRACTS,* 73069-13-3
- *CHEMICAL ABSTRACTS,* 73069-14-4
- **HALL MJ ; MIDDLETON RF ; WESTMACOTT D.** The fractional inhibitory concentration (FIC) index as a measure of synergy. *Journal of Antimicrobial Chemotherapy,* 1983, vol. 11 (5), 427-433